# EUROPEAN PATENT SPECIFICATION

(11) **EP 2 825 113 B1**
(45) Date of publication and mention of the grant of the patent: **25.04.2018**
(21) Application number: 12871086.0
(22) Date of filing: 15.03.2012
(51) Int. Cl.: A61B 17/56

(54) **DEVICE FOR AMELIORATING JOINT CONDITIONS AND DISEASES**
VORRICHTUNG ZUR LINDERUNG VON GELENKLEIDEN UND -ERKRANKUNGEN
DISPOSITIF D'AMÉLIORATION D'ÉTATS ET DE MALADIES ARTICULAIRES

(43) Date of publication of application: 21.01.2015
(73) Proprietor: Subchondral Solutions, Inc., Los Gatos, CA 95032 (US)
(72) Inventor: DEE, Derek, Rancho Palos Verdes, CA 90275 (US)
(74) Representative: Clark, Jane Anne
(86) International application number: PCT/US2012/029291
(87) International publication number: WO 2013/137889

(56) References cited:
- EP-A2- 1 129 675
- WO-A1-2008/016862
- WO-A2-2010/144065
- US-A- 5 026 373
- US-A1- 2004 230 315
- US-A1- 2005 060 037
- US-A1- 2009 024 229
- US-A1- 2011 029 081
- US-B2- 7 608 105

## Description

### BACKGROUND

There are a variety of conditions and diseases that impair the integrity and function of human joints. Among these joint conditions and diseases are arthroses, chondromalacia patella, isolated chondral defect, juvenile idiopathic arthritis, ligamentous deficiency arthroses, osteoarthritis (degenerative arthritis or degenerative joint disease), osteonecrosis, osteochondritis dissecans, patellar instability, post-ligamentous injury arthritis, post-meniscectomy arthritis, post-meniscectomy arthroses, post-traumatic arthritis, rheumatoid arthritis and septic arthritis. The incidence of arthritides alone in the United States exceeds 20%, with higher rates among women as compared to men. Treatment of joint conditions and diseases includes surgery and the administration of therapeutic agents. However, none of these treatments ameliorate all of the joint conditions and diseases.

Therefore, there is a need for a new method for ameliorating joint conditions and diseases.
WO2010/144065 describes a biomaterial which can be used in treatment of osteochondral bone defects and particularly in the treatment of defects of bond covered with hyaline cartilage. The biomaterial comprises a body manufactured from a biocompatible material, grooves which enable advancing in the defect area on the body, a space in the body which increases the contact area with the surrounding tissue and at least one channel which is opened to the space and which enables the blood to be transferred into the medium and enables the toxic materials to be removed from the medium.
WO2008/016862 describes a graft retention tack that can include a post configured to receive a bone growth factor and a head configured to receive a cartilage growth factor.

### SUMMARY

The present invention is set out in the appended claims. According to one embodiment, there is provided a device for ameliorating joint conditions and diseases. The device comprises a) a first section comprising a joint-ward end, an opposing mating end, and a lateral wall extending between the joint-ward end and the mating end, where the first section further comprises a peripheral column partially forming the lateral wall of the first section, a central column, and three or more than three struts, each strut extending between and connecting the peripheral column and the central column, and each strut thereby supporting the central column, where the joint-ward end further comprises a plurality of fenestrations, where each fenestration is formed by a confluence of the peripheral column, the central column and two adjacent struts of the three or more than three struts, and where the first section further comprises a central aperture within and formed by the central column, and configured to mate with a driver, b) a second section comprising a mating end, an opposing leading end, and a lateral wall extending between the mating end and the leading end, where the lateral wall of the second section comprises threads.

In one embodiment, the device further comprises an axial length, and the axial length is between 5 mm and 30 mm. In another embodiment, the device further comprises an axial length, and the axial length is between 5 mm and 20 mm. In another embodiment, the device further comprises an axial length, and the axial length is between 8 mm and 16 mm. In one embodiment, the first section further comprises a diameter between 5 mm and 30 mm. In another embodiment, the first section further comprises a diameter between 5 mm and 20 mm. In another embodiment, the first section further comprises a diameter between 8 mm and 16 mm. In another embodiment, the first section further comprises an axial length between 1 mm and 2 mm.

In one embodiment, each fenestration comprises a pear or teardrop shape. In another embodiment, one or more than one fenestration comprises a different size, different shape or both a different size and a different shape than one or more than one other fenestration.

In one embodiment, the central aperture comprises a six-pointed star shape. In another embodiment, the central aperture is round and comprises threads. In one embodiment, the peripheral column comprises one or more than one notch.

In one embodiment, the joint-ward end comprises a convex profile as seen on a cross-sectional, lateral perspective view. In another embodiment, the joint-ward end comprises a concave profile as seen on a cross-sectional, lateral perspective view. In another embodiment, the joint-ward end comprises a straight profile as seen on a cross-sectional, lateral perspective view. In one embodiment, the joint-ward end comprises a radius of curvature of between 20 mm and 50 mm. In another embodiment, the joint-ward end comprises a radius of curvature of between 15 mm and 45 mm. In another embodiment, the lateral wall of the first section comprises a generally convex profile as seen on a cross-sectional, lateral perspective view.

In one embodiment, the second section further comprises an axial length between 5 mm and 30 mm. In another embodiment, the second section further comprises an axial length between 5 mm and 20 mm. In another embodiment, the second section further comprises an axial length between 6 mm and 15 mm. In one embodiment, the lateral wall of the second section is generally cylindrical. In another embodiment, the lateral wall of the second section is generally conical, tapering between the mating end and the leading end. In one embodiment, the lateral wall of the second section tapers between 0.2 degrees and 15 degrees. In another embodiment, the lateral wall of the second section tapers between 1 degrees and 5 degrees. In another embodiment, the lateral wall of the second section tapers between 1 degrees and 3 degrees.

In one embodiment, the mating end of the first section and the mating end of the second section mate by a biocompatible adhesive. In another embodiment, the mating end of the first section and the mating end of the second section mate by a mating mechanism that is reversible. In another embodiment, the mating end of the first section and the mating end of the second section mate by a reversible twist locking mechanism. In another embodiment, the first section and the second section are made as a unified whole.

In one embodiment, the leading end comprises a scalloped edge. In another embodiment, the leading end comprises bevels. In another embodiment, the leading end comprises both a scalloped edge and bevels.

In one embodiment, the lateral wall of the second section further comprises a plurality of fenestrations between the threads. In another embodiment, the device further comprises a plurality of fenestrations formed by a confluence of the mating end of the first section and the mating end of the second section. In one embodiment, the device further comprises an insert, where the insert comprises a base and three or more than three extensions connected to the base and arranged radially around the base, and where each of the three or more than three extensions is configured to fit within a corresponding fenestration of the joint-ward end of first section of the device. In one embodiment, the insert further comprises porous biological material impregnated with matrix-promoting substances or serves as a scaffold for progenitor cells, or comprises both porous biological material impregnated with matrix-promoting substances and serves as a scaffold for progenitor cells.

According to another embodiment , there is provided a method for ameliorating a joint condition or disease in a patient. The method comprises a) identifying a patient with a joint condition or disease that is suitable for treatment by the method, where the joint comprises a bone with a surface comprising a defect caused by the joint condition or disease, b) accessing the joint, c) placing a guidepin within the center of the defect, d) creating a space in the defect of the bone, e) providing a first device according to an embodiment, f) attaching the first device to a driver by mating the distal end of the driver with the central aperture of the first device, and g) screwing the first device into the space using the driver until the joint-ward end of the first device forms a shape that substantially recreates the shape of a normal articulation surface on the bone after implantation.

In one embodiment, the joint is a diarthrodial joint. In another embodiment, the joint is selected from the group consisting of an acetabulofemoral joint, an acromioclavicular joint, a femoropatellar joint, a femorotibial joint, a glenohumeral joint, a humeroradial joint, a humeroulnar joint, an interphalangeal joint, a metacarpal joint, a radioulnar joint and a talocrural joint. In one embodiment, the patient is a human. In one embodiment, the patient is a non-human animal. In one embodiment, the joint condition and disease is selected from the group consisting of arthroses, chondromalacia patella, isolated chondral defect, juvenile idiopathic arthritis, ligamentous deficiency arthroses, osteoarthritis (degenerative arthritis or degenerative joint disease), osteonecrosis, osteochondritis dissecans, patellar instability, post-ligamentous injury arthritis, post-meniscectomy arthritis, post-meniscectomy arthroses, post-traumatic arthritis, rheumatoid arthritis and septic arthritis.

In one embodiment, identifying the patient comprises diagnosing the patient with a joint condition and disease. In another embodiment, diagnosing the patient comprises performing one or more than one of action selected from the group consisting of performing a physical examination, performing a non-invasive imaging examination and performing arthroscopy. In one embodiment, identifying the patient comprises consulting patient records to determine if the patient has a joint condition or disease suitable for treatment by the method. In one embodiment, accessing the joint is accomplished by arthroscopy. In one embodiment, the joint is accomplished by an open surgical procedure.

In one embodiment, the surface of the bone comprises one or more than one abnormality, and the method further comprises using a burr, or a suction shaver, or both a burr and a suction shaver to remove some or all of the one or more than one abnormality thereby creating a smoother articulation surface. In one embodiment, the method further comprises creating one or more than one vascular channel in the bone deep to the space using a drill bit guide positioned over the guidepin and a drill bit passed within the drill bit guide. In another embodiment, the method further comprises injecting a biological material into the first device. In one embodiment, the method further comprises placing an insert in the first device. In one embodiment, the method further comprises placing one or more than one additional device in the defect.

### DRAWINGS

These and other features, aspects and advantages of the present invention will become better understood with regard to the following description, appended claims, and accompanying figures where:
Figure 1 is a lateral perspective view of one embodiment of a device for ameliorating joint conditions and diseases according to the present invention;
Figure 2 is a top, lateral perspective view of another embodiment of a device for ameliorating joint conditions and diseases according to the present invention;
Figure 3 is an exploded, top, lateral perspective view of the embodiment of the device for ameliorating joint conditions and diseases shown in Figure 1;
Figure 4 is a top perspective view of the embodiment of the device for ameliorating joint conditions and diseases shown in Figure 1;
Figure 5 is a bottom perspective view of the embodiment of the device for ameliorating joint conditions and diseases shown in Figure 1;
Figure 6 is a top perspective view of the embodiment of the device for ameliorating joint conditions and diseases shown in Figure 2;
Figure 7 is a top perspective view of another embodiment of the device for ameliorating joint conditions and diseases according to the present invention;
Figure 8 is a top perspective view of another embodiment of the device for ameliorating joint conditions and diseases according to the present invention;
Figure 9 is a cross-sectional, lateral perspective view of the embodiment of the device for ameliorating joint conditions and diseases shown in Figure 1 taken along line 9-9;
Figure 10 is a cross-sectional, lateral perspective view of another embodiment of the device for ameliorating joint conditions and diseases according to the present invention;
Figure 11 is a cross-sectional, lateral perspective view of the embodiment of the device for ameliorating joint conditions and diseases shown in Figure 2 taken along line 11-11;
Figure 12 is a top, lateral perspective view of one embodiment of an insert according to the present invention for use with a device for ameliorating joint conditions and diseases according to the present invention;
Figure 13 is a bottom, lateral perspective view of the embodiment of the insert shown in Figure 12;
Figure 14 is a top, lateral perspective view of one embodiment of the device for ameliorating joint conditions and diseases shown in Figure 1 with the insert shown in Figure 12 according to the present invention affixed to the device;
Figure 15 is a cross-sectional view of the device for ameliorating joint conditions and diseases shown in Figure 1 with the insert shown in Figure 12 according to the present invention affixed to the device; and
Figure 16 through Figure 35 are schematic depictions or some steps of a method for ameliorating joint conditions and diseases according to the present invention.

### DESCRIPTION

Described herein is a device for ameliorating joint conditions and diseases. Also described herein is
a method for ameliorating a joint condition or disease in a patient. The method may comprise providing a device as described herein. The device and methods will now be disclosed in detail.

As used in this disclosure, except where the context requires otherwise, the term "comprise" and variations of the term, such as "comprising," "comprises" and "comprised" are not intended to exclude other additives, components, integers or steps.

As used in this disclosure, except where the context requires otherwise, the method steps disclosed and shown are not intended to be limiting nor are they intended to indicate that each step is essential to the method or that each step must occur in the order disclosed but instead are exemplary steps only.

All dimensions specified in this disclosure are by way of example only and are not intended to be limiting, except where the context requires otherwise. Further, the proportions shown in these Figures are not necessarily to scale. As will be understood by those with skill in the art with reference to this disclosure, the actual dimensions and proportions of any device or part of a device disclosed in this disclosure will be determined by its intended use.

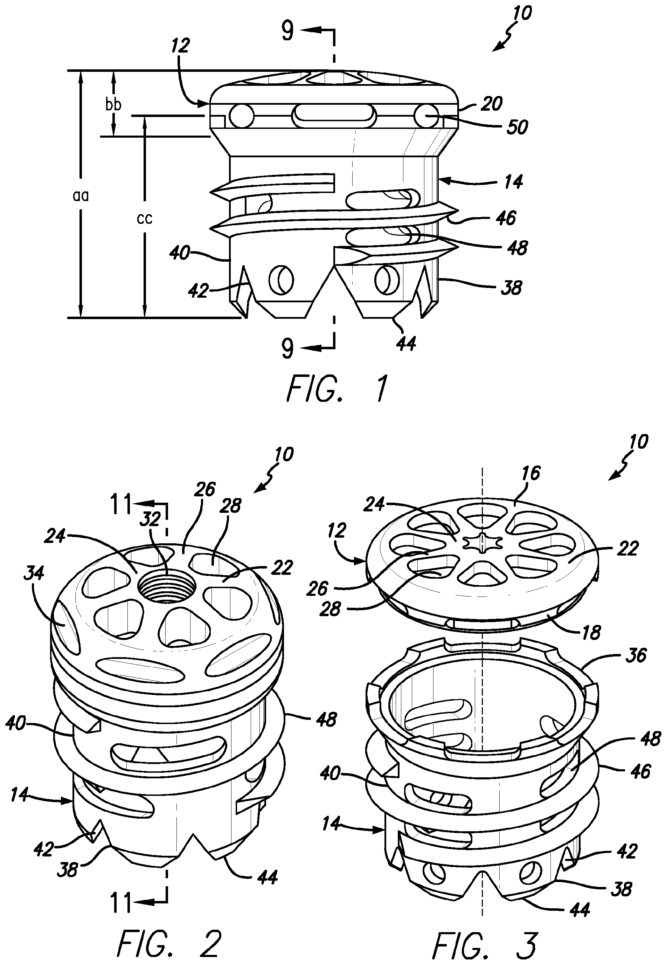

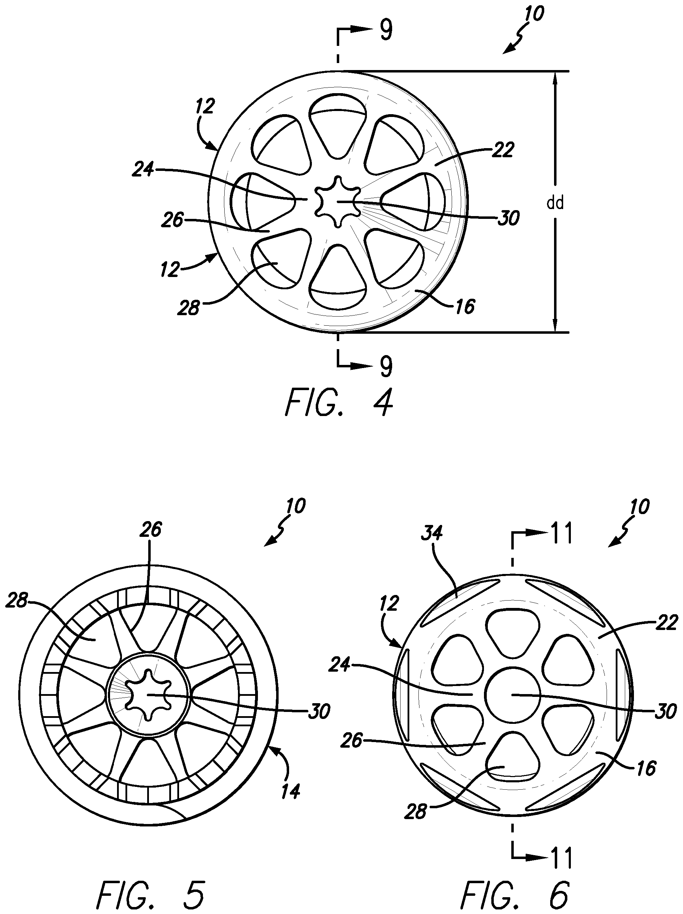

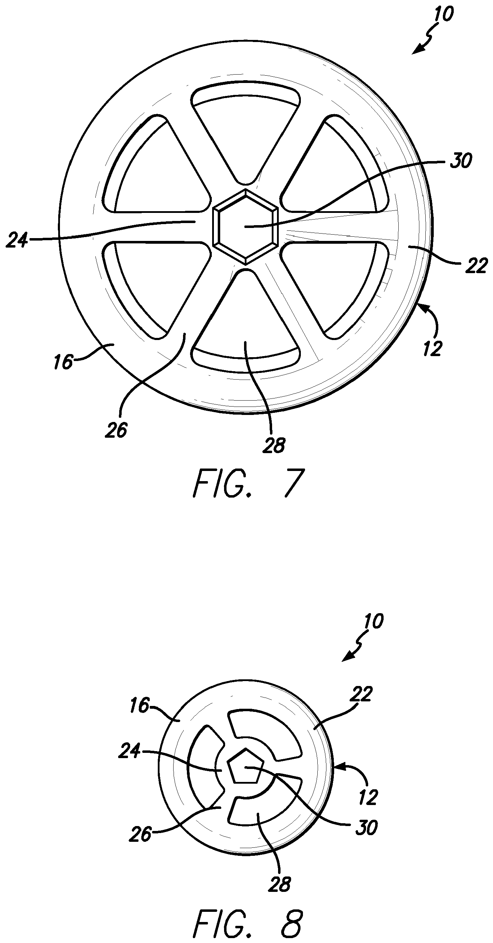

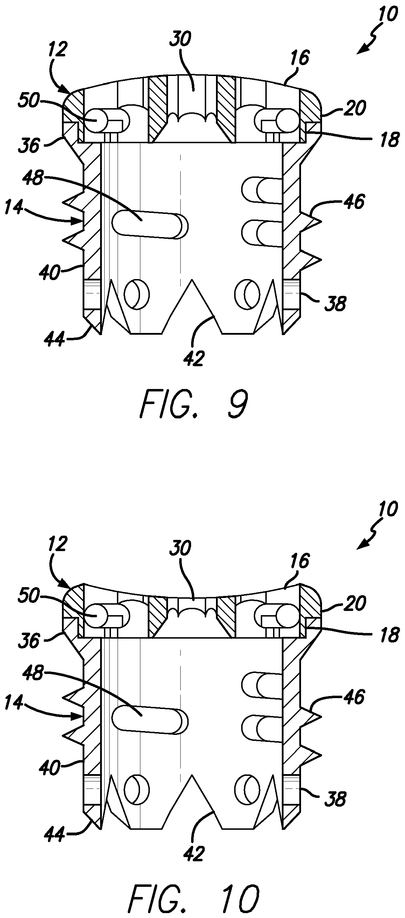

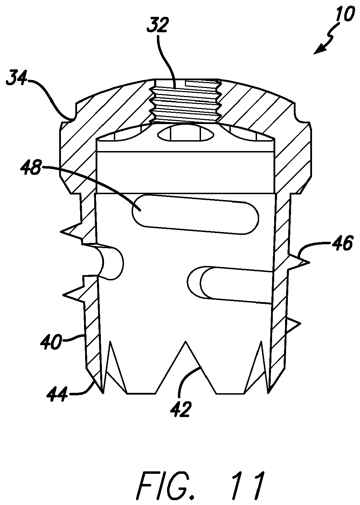

According to one embodiment of the present invention, there is provided a device for ameliorating joint conditions and diseases. Referring now to Figure 1 through Figure 11, there are shown, respectively, a lateral perspective view of one embodiment of a device for ameliorating joint conditions and diseases according to the present invention (Figure 1); a top, lateral perspective view of another embodiment of a device for ameliorating joint conditions and diseases according to the present invention (Figure 2); an exploded, top, lateral perspective view of the embodiment of the device for ameliorating joint conditions and diseases shown in Figure 1 (Figure 3); a top perspective view of the embodiment of the device for ameliorating joint conditions and diseases shown in Figure 1 (Figure 4); a bottom perspective view of the embodiment of the device for ameliorating joint conditions and diseases shown in Figure 1 (Figure 5); a top perspective view of the embodiment of the device for ameliorating joint conditions and diseases shown in Figure 2 (Figure 6); a top perspective view of another embodiment of the device for ameliorating joint conditions and diseases according to the present invention (Figure 7); a top perspective view of another embodiment of the device for ameliorating joint conditions and diseases according to the present invention (Figure 8); a cross-sectional, lateral perspective view of the embodiment of the device for ameliorating joint conditions and diseases shown in Figure 1 taken along line 9-9 (Figure 9); a cross-sectional, lateral perspective view of another embodiment of the device for ameliorating joint conditions and diseases according to the present invention (Figure 10); and a cross-sectional, lateral perspective view of the embodiment of the device for ameliorating joint conditions and diseases shown in Figure 2 taken along line 11-11 (Figure 11). As can be seen, the device 10 comprises a first section 12 and a second section 14, and comprises a generally cylindrical shape partially or completely closed at one end. The device 10 further comprises an axial length (a-a). In one embodiment, the axial length (a-a) is between 5 mm and 30 mm. In another embodiment, the axial length (a-a) is between 5 mm and 20 mm. In another embodiment, the axial length (a-a) is between 8 mm and 16 mm. In a preferred embodiment, the axial length (a-a) is 8 mm. In another preferred embodiment, the axial length (a-a) is 12 mm. In another preferred embodiment, the axial length (a-a) is 16 mm.

The first section 12 of the device 10 comprises a joint-ward end 16, an opposing mating end 18, and a lateral wall 20 extending between the joint-ward end 16 and the mating end 18. The first section 12 further comprises a diameter (d-d) and an axial length (b-b). In one embodiment, the diameter (d-d) is between 5 mm and 30 mm. In another embodiment, the diameter (d-d) is between 5 mm and 20 mm. In another embodiment, the diameter (d-d) is between 8 mm and 16 mm. In a preferred embodiment, the diameter (d-d) is 8 mm. In another preferred embodiment, the diameter (d-d) is 12 mm. In another preferred embodiment, the diameter (d-d) is 16 mm. In one embodiment, the axial length (b-b) is between 0.5 mm and 2.5 mm. In another embodiment, the axial length (b-b) is between 1 mm and 2 mm. In a preferred embodiment, the axial length (b-b) is 1.25 mm.

In one embodiment, the first section 12 further comprises a peripheral column 22 partially forming the lateral wall 20, a central column 24, and three or more than three struts 26, each strut 26 extending between and connecting the peripheral column 22 and the central column 24, and each strut 26 thereby supporting the central column 24.

In one embodiment, the joint-ward end 16 further comprises a plurality of fenestrations 28, where each fenestration 28 is formed by a confluence of the peripheral column 22, the central column 24, and two adjacent struts 26 of the three or more than three struts 26. Each fenestration 28 can comprise any shape suitable for the intended purpose of the device 10, as will be understood by those with skill in the art with respect to this disclosure. In one embodiment, as shown particularly in Figure 2, Figure 3, Figure 4, Figure 5, Figure 6 and Figure 7, each fenestration 28 comprises a pear or teardrop shape. In another embodiment, as shown in Figure 8, each fenestration 28 comprises a kidney shape. In another embodiment, each fenestration 28 comprises an oval or a round shape. As will be understood by those with skill in the art with respect to this disclosure, all fenestrations 28 on the device 10 can comprise the same size and shape or one or more than one fenestration 28 can comprise a different size, different shape or both a different size and a different shape than one or more than one other fenestration 28. In another embodiment, the joint-ward end 16 can be solid between the central column 24 and the peripheral column 22.

The first section 12 further comprises a central aperture 30 within and formed by the central column 24. The central aperture 30 can extend axially completely through the joint-ward end 16 as shown particularly in Figure 9, Figure 10 and Figure 11, or can be blind-ended extending only partially through within joint-ward end 16. The central aperture 30 is configured to mate with a driver as disclosed below. The central aperture 30 comprises any shape suitable for the intended purpose of the device 10, as will be understood by those with skill in the art with respect to this disclosure. In one embodiment, the central aperture 30 comprises a square shape. In one embodiment, as shown in Figure 2 and Figure 6, the central aperture 30 comprises a round shape. In another embodiment, as shown in Figure 3, Figure 4 and Figure 5, the central aperture 30 comprises a six-pointed star shape. In another embodiment, as shown in Figure 7, the central aperture 30 comprises a pentagonal shape. In another embodiment, as shown in Figure 8, the central aperture 30 comprises a hexagonal shape. In one embodiment, as shown in Figure 2 and Figure 11, the central aperture 30 comprises threads 32 to assist in mating with a driver.

In one embodiment, peripheral column 22 of the first section 12 comprises one or more than one notch 34 as seen in Figure 2, Figure 6 and Figure 11. The one or more than one notch can be used to mate with a driver in addition to the central aperture 30 or instead of the central aperture 30, as will be understood by those with skill in the art with respect to this disclosure.

The joint-ward end 16 of the first section 12 of the device 10 performs a partial load-bearing function after implantation, and comprises a shape suitable to substantially match the shape of the articulation surface that the device 10 recreates on the bone after implantation, as will be understood by those with skill in the art with respect to this disclosure. Therefore, the joint-ward end 16 can have either a convex profile as seen on a cross-sectional, lateral perspective view, as shown in Figure 9 and Figure 11, a concave profile as seen on a cross-sectional, lateral perspective view, as shown in Figure 10, or a straight profile as seen on a cross-sectional, lateral perspective view. In one embodiment, the joint-ward end has a convex profile having a radius of curvature of between 10 mm and 50 mm. In another embodiment, the joint-ward end has a convex profile as seen on a cross-sectional, lateral perspective view with a radius of curvature of between 15 mm and 45 mm. In another embodiment, the joint-ward end has a convex profile as seen on a cross-sectional, lateral perspective view with a radius of curvature of between 20 mm and 30 mm. In one embodiment, the joint-ward end has a concave profile as seen on cross-sectional, lateral perspective view with a radius of curvature of between 10 mm and 50 mm. In another embodiment, the joint-ward end has a concave profile as seen on cross-sectional, lateral perspective view with a radius of curvature of between 15 mm and 45 mm. In another embodiment, the joint-ward end has a concave profile as seen on cross-sectional, lateral perspective view with a radius of curvature of between 20 mm and 30 mm. In a preferred embodiment, the joint-ward end 16 comprises a smooth surface facing the center of joint after implantation, as will be understood by those with skill in the art with respect to this disclosure. In a preferred embodiment, the joint-ward end 16 is polished to make the surface smooth.

The lateral wall 20 of the first section 12 can be any shape suitable for the intended purpose of the device 10, as will be understood by those with skill in the art with respect to this disclosure. In a preferred embodiment, the lateral wall 20 of the first section 12 comprises a generally convex profile as seen on a cross-sectional, lateral perspective view, as shown in Figure 9 and Figure 11. This convex profile advantageously provides a smooth transition to and encourages biologic bonding to surrounding cartilage and bone after implantation, as will be understood by those with skill in the art with respect to this disclosure.

The device 10 further comprises a second section 14. The second section 14 of the device 10 comprises a mating end 36, an opposing leading end 38, and a lateral wall 40 extending between the mating end 36 and the leading end 38. The second section 14 further comprises an axial length (c-c). In one embodiment, the axial length (c-c) is between 5 mm and 30 mm. In another embodiment, the axial length (c-c) is between 5 mm and 20 mm. In another embodiment, the axial length (c-c) is between 6 mm and 15 mm. In a preferred embodiment, the axial length (c-c) is 6 mm. In another preferred embodiment, the axial length (c-c) is 10 mm. In another preferred embodiment, the axial length (c-c) is 15 mm. In one embodiment, the lateral wall 40 of the second section 14 is generally cylindrical as seen in Figure 1, Figure 9 and Figure 10. In another embodiment, the lateral wall 40 of the second section 14 is generally conical, tapering between the mating end 36 and the leading end 38 as seen in Figure 11. In one embodiment, the lateral wall 40 of the second section 14 tapers between 0.2 degrees and 15 degrees. In another embodiment, the lateral wall 16 tapers between 1 degrees and 5 degrees. In another embodiment, the lateral wall 40 of the second section 14 tapers between 1 degrees and 3 degrees.

The mating end 36 of the second section 14 of the device 10 is configured to mate with the mating end 18 of the first section 12 of the device 10. The mating end 18 of the first section 12 and the mating end 36 of the second section 14 can comprise any mating mechanism suitable for the intended purpose of the device 10 can be used, as will be understood by those with skill in the art with respect to this disclosure. In one embodiment, the mating end 18 of the first section 12 and the mating end 36 of the second section 14 mate by a suitable biocompatible adhesive, as will be understood by those with skill in the art with respect to this disclosure. In a preferred embodiment, the mating mechanism is reversible, allowing an interchange of an alternate first section 12 to a specific second section 14 so that the device 10 can be reconfigured as needed for contouring to a particular joint surface, thereby decreasing the number of second sections 14 that need to be stored on site, as will be understood by those with skill in the art with respect to this disclosure. In one embodiment, the mating end 18 of the first section 12 and the mating end 36 of the second section 14 mate by a reversible twist locking mechanism, as will be understood by those with skill in the art with respect to this disclosure. In another embodiment, the first section 12 and the second section 14 are made as a unified whole as shown in Figure 11 and are not separable.

The leading end 38 of the second section 14 of the device 10 is configured to place the device 10 into a prepared space made according to a method according to the present invention. In one embodiment, the leading end 38 comprises a scalloped edge 42. In another embodiment, the leading end 38 comprises bevels 44. In a preferred embodiment, the leading end 38 comprises both a scalloped edge 42 and bevels 44 as shown particularly in Figure 1, Figure 3, Figure 5 and Figure 6.

The lateral wall 40 of the second section 14 of the device 10 extends between the mating end 36 and the leading end 38. The lateral wall 40 of the second section 14 comprises threads 46 for anchoring the device 10 within the bone. In one embodiment, the lateral wall 40 of the second section 14 further comprises a plurality of fenestrations 48 between the threads 46. In a preferred embodiment, the device 10 further comprises a plurality of fenestrations 50 formed by a confluence of the mating end 18 of the first section 12 and the mating end 36 of the second section 14. Each fenestration 48, 50 can comprise any shape suitable for the intended purpose of the device 10, as will be understood by those with skill in the art with respect to this disclosure. In a preferred embodiment, each fenestration 48, 50 is oval or round. In one embodiment, the lateral wall 40 of the second section 14 is textured to promote bony ingrowth after implantation, as will be understood by those with skill in the art with respect to this disclosure.

The first section 12 and the second section 14 can comprise any material suitable for the intended purpose of the device 10, as will be understood by those with skill in the art with respect to this disclosure. In one embodiment, the first section 12 comprises a material selected from the group consisting of a biocompatible plastic, a biocomposite polymer, a metal and a metal alloy. In one embodiment, the first section 12 comprises a material selected from the group consisting of carbon fiber, cobalt chrome, nitinol, polycaprolactone (PCL), polyether-ether-ketone (PEEK), tantalum and titanium. In one embodiment, the second section 14 comprises a material selected from the group consisting of a biocompatible plastic, a biocomposite polymer, a metal and a metal alloy. In one embodiment, the second section 14 comprises a material selected from the group consisting of carbon fiber, cobalt chrome, nitinol, polycaprolactone (PCL), polyether-ether-ketone (PEEK), tantalum and titanium. In one embodiment, the first section 12 comprises a first material and the second section 14 comprises a second material, where the first material and the second material are the same material. In another embodiment, the first section 12 comprises a first material and the second section 14 comprises a second material, where the first material and the second material are the different materials.

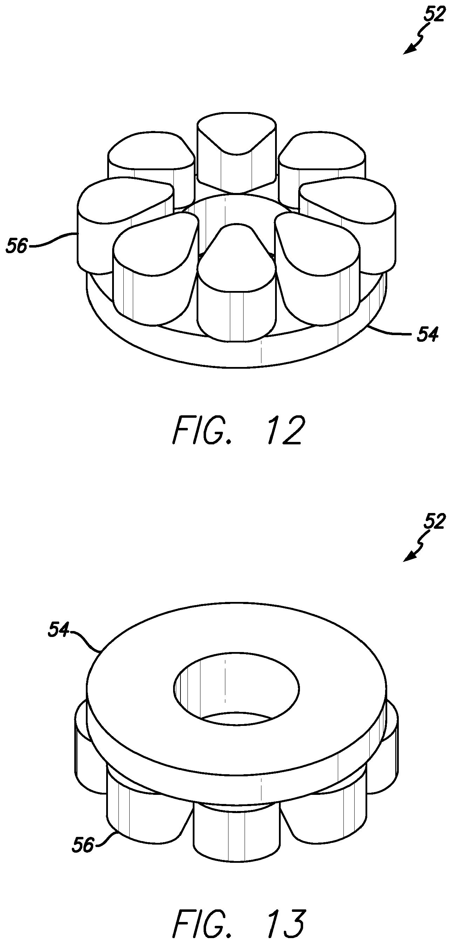

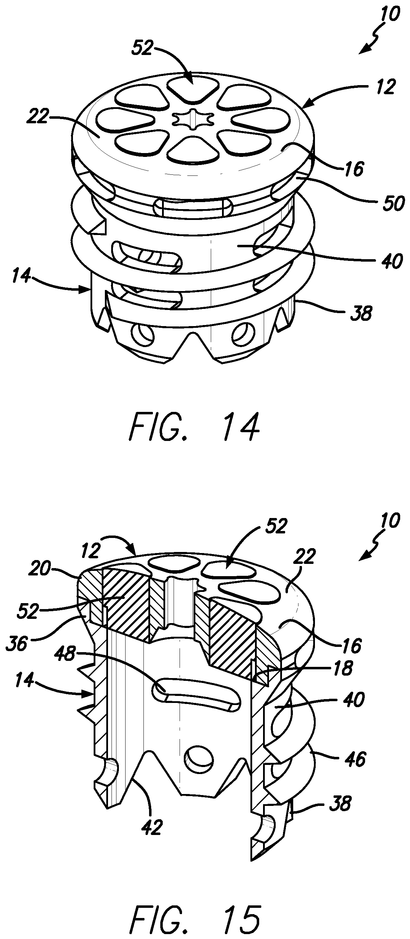

In one embodiment, the device 10 further comprises an insert 52. Referring now to Figure 12, Figure 13, Figure 14 and Figure 15, there are shown, respectively, a top, lateral perspective view of one embodiment of an insert according to the present invention for use with a device for ameliorating joint conditions and diseases according to the present invention (Figure 12); a bottom, lateral perspective view of the embodiment of the insert shown in Figure 12 (Figure 13); a top, lateral perspective view of one embodiment of the device for ameliorating joint conditions and diseases shown in Figure 1 with the insert shown in Figure 12 according to the present invention affixed to the device (Figure 14); and a cross-sectional view of the device for ameliorating joint conditions and diseases shown in Figure 1 with the insert shown in Figure 12 (Figures 15). As can be seen, the insert 52 comprises a base 54 and three or more than three extensions 56 connected to the base 54 and arranged radially around the base 54. Each of the three or more than three extensions 56 is configured to fit within a corresponding fenestration 28 of the joint-ward end 16 of the first section 12 of the device 10, such that when the insert 52 is mated to the first section 12 of the device 10, the insert 52 occupies each of the three or more than three fenestrations 28 as shown particularly in Figure 5. The insert 52 comprises porous biological material impregnated with matrix-promoting substances or serves as a scaffold for progenitor cells, or comprises both porous biological material impregnated with matrix-promoting substances and serves as a scaffold for progenitor cells.

The device 10 can be made by any suitable method, as will be understood by those with skill in the art with respect to this disclosure. In one embodiment, the first section 12 and the second section 14 are machined from modular parts such as by direct metal laser sintering, as will be understood by those with skill in the art with respect to this disclosure.

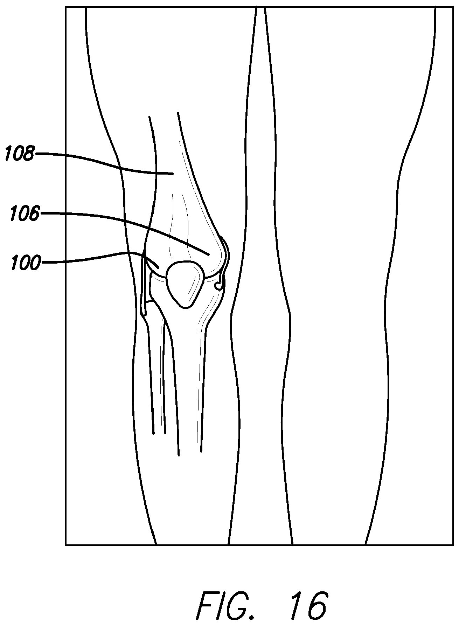

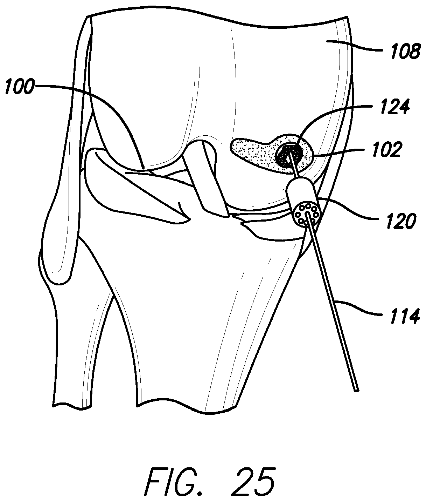

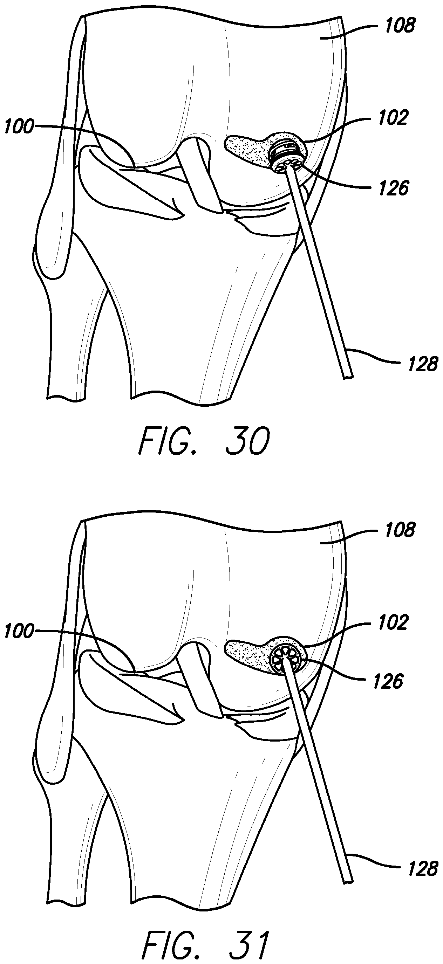

A method is provided
for ameliorating a joint condition or disease in a patient. Referring now to Figure 16 through Figure 35, there are shown schematic depictions of some steps of a method for ameliorating joint conditions and diseases. The Figures show the embodiment of the method being used on a femorotibial joint 100 to ameliorate an arthritic condition which has caused a defect 102 on an articulation surface 104 of a bone or joint, shown here as on the medial condyle 106 of the femur 108.

The method comprises identifying a patient with a joint condition or disease that is suitable for treatment by the present method, where the joint comprises a bone with a surface comprising a defect caused by the joint condition or disease. As will be understood by those with skill in the art with respect to this disclosure, the joint can be any joint with a hyaline cartilage bearing surface, joint capsule, and synovial fluid. In one embodiment, the joint is a diarthrodial joint (also know as a synovial joint). In one embodiment, the joint is selected from the group consisting or an acetabulofemoral joint, an acromioclavicular joint, a femoropatellar joint, a femorotibial joint, a glenohumeral joint, a humeroradial joint, a humeroulnar joint, an interphalangeal joint, a metacarpal joint, a radioulnar joint and a talocrural joint. In one embodiment, the patient is a human. In one embodiment, the patient is a non-human animal. In a preferred embodiment, the joint condition and disease is selected from the group consisting of arthroses, chondromalacia patella, isolated chondral defect, juvenile idiopathic arthritis, ligamentous deficiency arthroses, osteoarthritis (degenerative arthritis or degenerative joint disease), osteonecrosis, osteochondritis dissecans, patellar instability, post-ligamentous injury arthritis, post-meniscectomy arthritis, post-meniscectomy arthroses, post-traumatic arthritis, rheumatoid arthritis and septic arthritis. In one embodiment, identifying the patient comprises diagnosing the patient with a joint condition and disease. In one embodiment, diagnosing the patient comprises performing one or more than one of action selected from the group consisting of performing a physical examination, performing a non-invasive imaging examination (such as magnetic resonance imaging, computerized tomography and ultrasound) and performing arthroscopy. In another embodiment, identifying the patient comprises consulting patient records to determine if the patient has a joint condition or disease suitable for treatment by the present method.

Next, the method further comprises accessing the joint 100. In one embodiment, accessing the joint 100 is accomplished by arthroscopy. In another embodiment, accessing the joint 100 is accomplished by an open surgical procedure, such as for example a mini-open procedure.

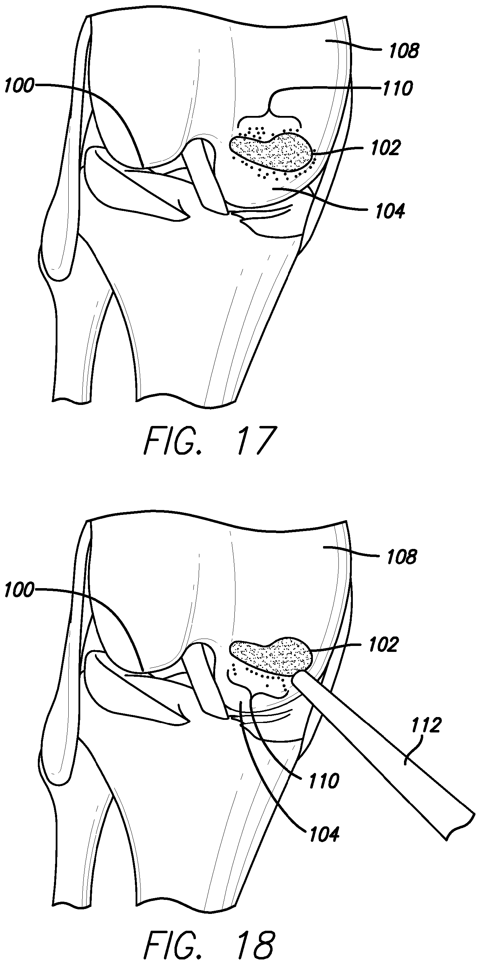

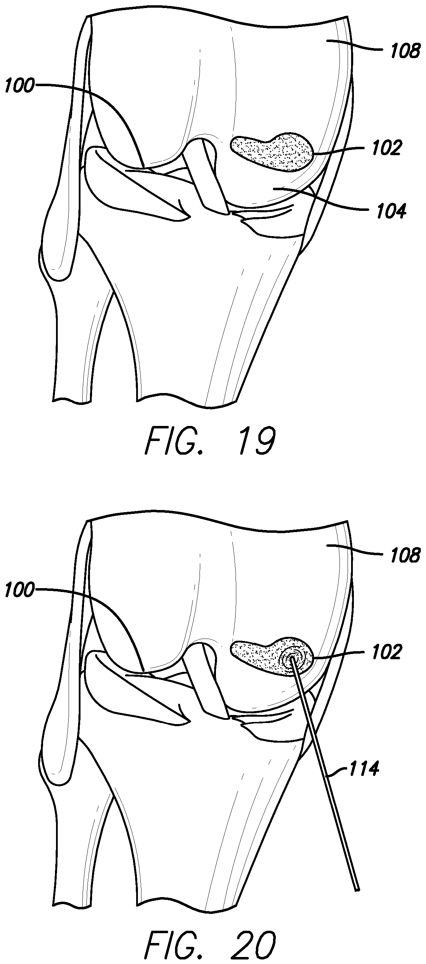

In one embodiment, as shown in Figure 17 and Figure 18, the surface 104 of the bone comprises an abnormality 110 (such as for example area cartilage softening, thinning, damage, or absence), and the method further comprises using a burr, or a suction shaver, or both a burr and a suction shaver 112 to remove some or all of the abnormalities 110 thereby creating a smoother articulation surface 104 as shown in Figure 19.

Then, the method further comprises placing a guidepin 114 within the center of the defect 102 as shown in Figure 20.

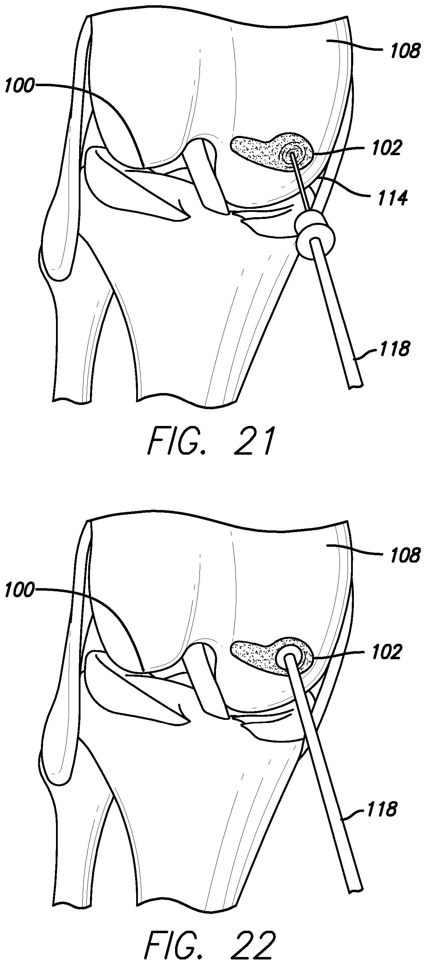

Next, the method further comprises creating a space 116 in the defect 102 of the bone for a device. In one embodiment, the space 116 is created using a bone reamer 118 placed over the guidepin 114 to core and plane the surface of the defect 102 as shown in Figure 21, Figure 22 and Figure 23. The bone reamer 118 is then removed leaving the guidepin 114 in place.

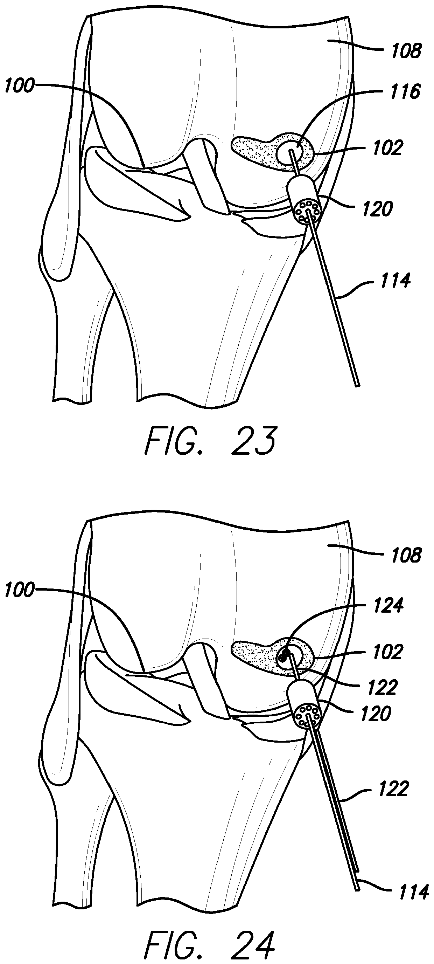

In one embodiment, the method further comprises creating one or more than one vascular channel in the bone deep to the space 116 using a drill bit guide 120 positioned over the guidepin 114 and a drill bit 122 passed within the drill bit guide 120 as shown in Figure 23, Figure 24 and Figure 25. Confirmation of creation of the one or more than one vascular channel is made by the presence of blood 124 leaking into the space 116 from the one or more than one vascular channel. The drill bit guide 120 and drill bit 122 are then removed leaving the guidepin 114 in place.

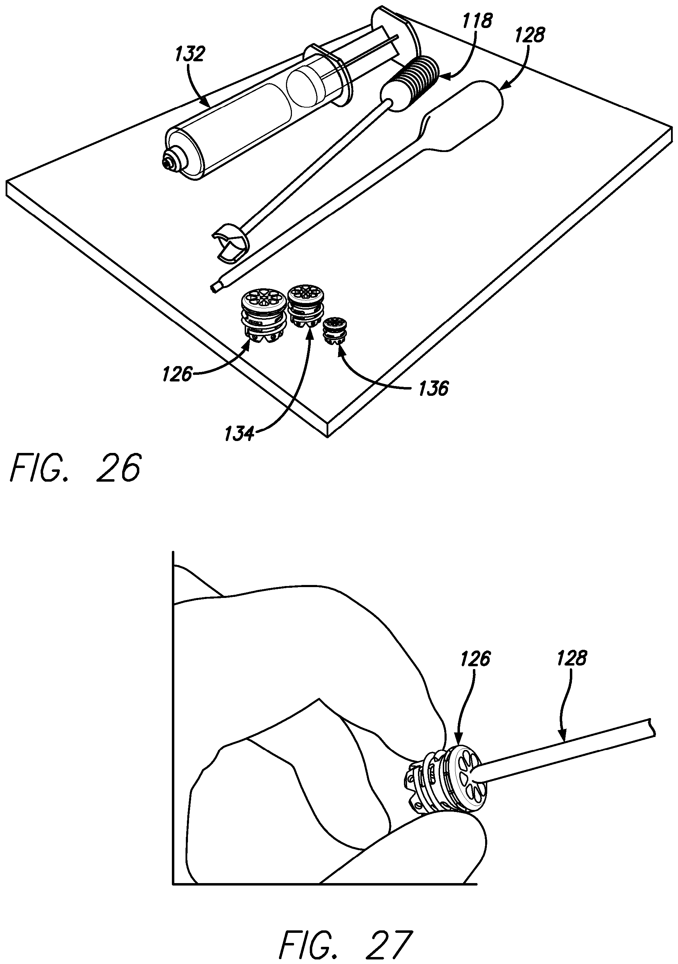

Next, the method further comprises providing a first device 126 for ameliorating joint conditions and diseases suitable for ameliorating the joint condition or disease of the patient as can be seen in Figure 26. In one embodiment, the first device 126 is a device according to the present invention. The first device 126 provided has a size suitable for incorporation into the space 116 made in the defect 102, and the joint-ward end of the first device 126 comprises a shape suitable to substantially match the shape of the articulation surface 104 that the first device 126 recreates on the bone after implantation, as will be understood by those with skill in the art with respect to this disclosure. Referring now to Figure 27, the first device 126 is attached to a driver 128, such as for example by mating the distal end of the driver 128 with the central aperture of the first device 126.

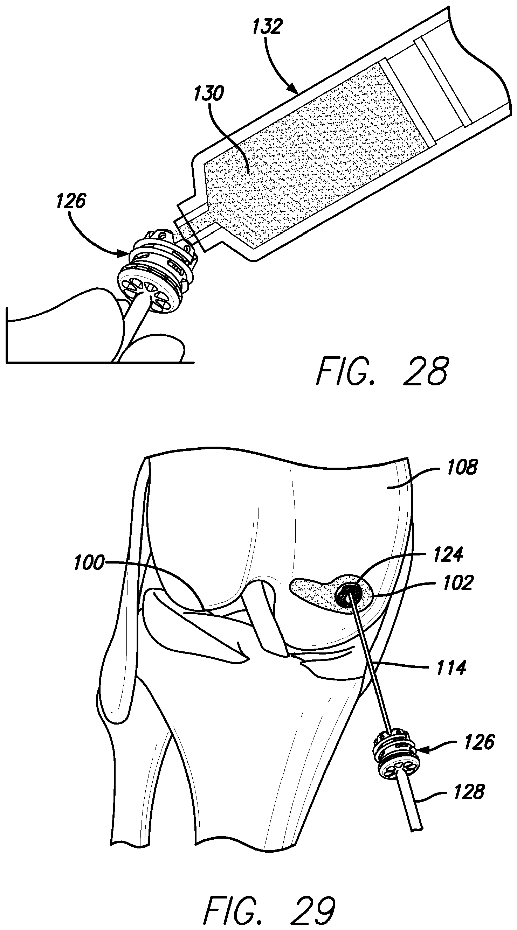

In one embodiment, the method further comprises injecting a biologic material, such as for example stem cells or platelet rich plasma, or both stem cells and platelet rich plasma 130 into the first device 126 using an injector 132 as shown in Figure 28. In one embodiment, the method further comprises placing an insert according to the present invention in the first device 126 instead of injecting a biocompatible bone cement in the first device 126. In one embodiment, the insert is a biological material according to the present invention.

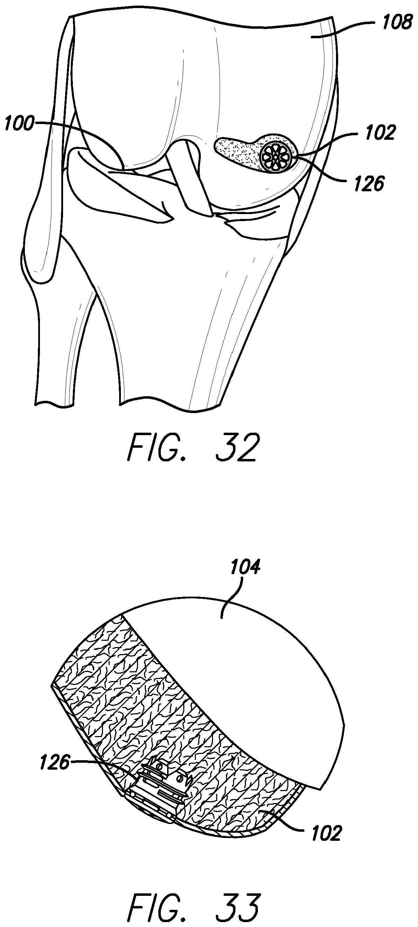

Then, the method further comprises screwing the first device 126 into the space 116 using the driver 128, as shown in Figure 29, Figure 30, Figure 31 and Figure 32. Figure 33 is a partial, lateral cross-section of the medial condyle 106 at the site of the defect 102 showing placement of the first device 126. As can be seen, the joint-ward end of the first device 126 forms a shape that substantially recreates the shape of a normal articulation surface on the bone after implantation.

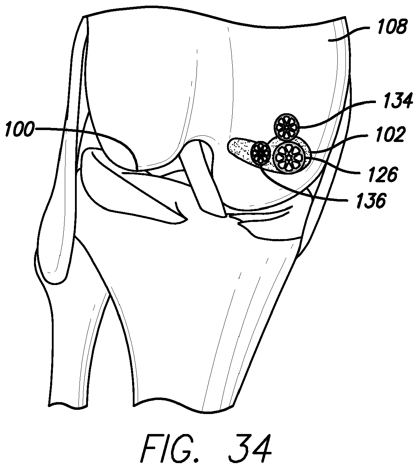

In one embodiment, as can be seen in Figure 26 and Figure 34, the method further comprises placing one or more than one additional device 134, 136 in the defect 102. In one embodiment, the one or more than one additional device is one additional device. In another embodiment, one or more than one additional device is two additional devices. As will be understood by those with skill in the art with respect to this disclosure, the one or more than one additional device 134, 136 can be the same as the first device in terms of size and shape or can be different than the first device in terms of size and shape.

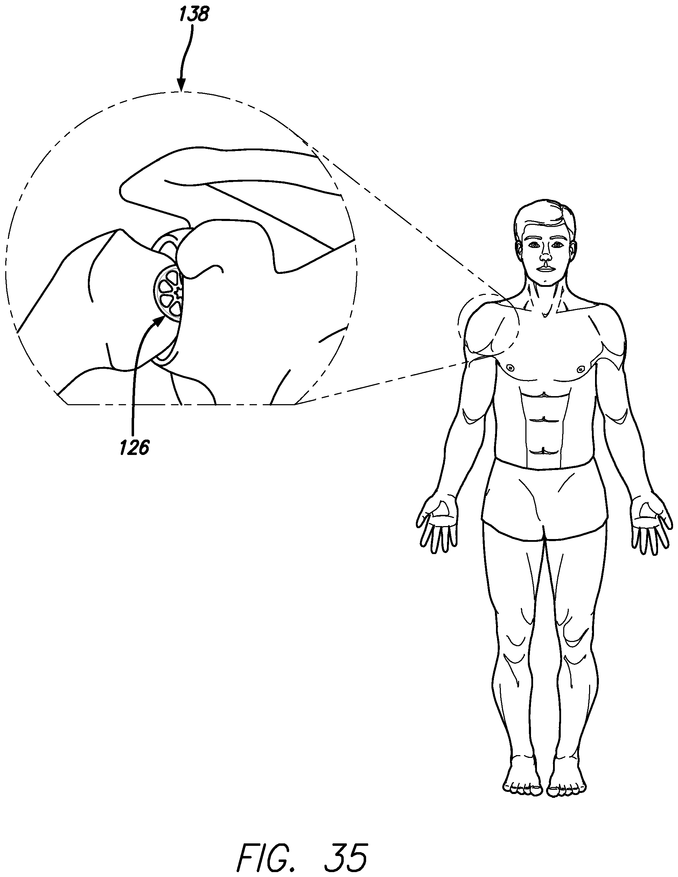

Though the method has been disclosed with respect to a defect 102 in a femorotibial joint 100, corresponding methods can be used with other joints. Figure 35 is a partial, lateral cross-section of a glenohumeral joint 138 at the site of a defect showing placement of a device for ameliorating joint conditions and diseases.

Although the present invention has been discussed in considerable detail with reference to certain preferred embodiments, other embodiments are possible. Therefore, the scope of the appended claims should not be limited to the description of preferred embodiments contained in this disclosure.

## Claims

1. A device for ameliorating joint conditions and diseases, the device comprising:
a) a first section (12) comprising a joint-ward end (16), an opposing mating end (18), and a lateral wall (20) extending between the joint-ward end (16) and the mating end (18);
where the first section (12) further comprises a peripheral column (22) partially forming the lateral wall (20) of the first section (12), a central column (24), and three or more than three struts (26), each strut extending between and connecting the peripheral column (22) and the central column (24), and each strut thereby supporting the central column (24);
where the joint-ward end (16) further comprises a plurality of fenestrations (28), where each fenestration is formed by a confluence of the peripheral column (22), the central column (24) and two adjacent struts (26) of the three or more than three struts (26); and
where the first section (12) further comprises a central aperture (30) within and formed by the central column (24); and
b) a second section (14) comprising a mating end (36), an opposing leading end (38), and a lateral wall (40) extending between the mating end (36) and the leading end (38); **characterised in that** the lateral wall (40) of the second section (14) comprises threads (46) and the central aperture within the central column is configured to mate with a driver (128).

2. The device of claim 1, further comprising an axial length, and where the axial length is between 5 mm and 30 mm.

3. The device of claim 1, where the first section (12) further comprises a diameter between 5 mm and 30 mm.

4. The device of claim 1, where one or more than one fenestration (28) comprises a different size, different shape or both a different size and a different shape than one or more than one other fenestration.

5. The device of claim 1, where the peripheral column (22) comprises one or more than one notch.

6. The device of claim 1, where the joint-ward end (16) comprises a convex profile, a concave profile or a straight profile as seen on a cross-sectional, lateral perspective view.

7. The device of claim 1, where the joint-ward end (16) comprises a radius of curvature of between about 10 mm and 50 mm.

8. The device of claim 1, where the lateral wall (20) of the first section (12) comprises a generally convex profile as seen on a cross-sectional, lateral perspective view, or where the second section further comprises an axial length between 5 mm and 30 mm.

9. The device of claim 1, where the lateral wall of the second section (14) is cylindrical or generally conical, tapering between the mating end and the leading end, optionally where the lateral wall of the second section tapers between 0.2 degrees and 15 degrees.

10. The device of claim 1, where the mating end (18) of the first section (12) and the mating end (36) of the second section (14) mate by a mating mechanism that is reversible, or
where the leading end (38) comprises a scalloped edge, a bevel, or a combination thereof.

11. The device of claim 1, where the lateral wall (40) of the second section (14) further comprises a plurality of fenestrations (48) between the threads (46); optionally wherein the plurality of fenestrations (48) are formed by a confluence of the mating end of the first section (12) and the mating end of the second section (14).

12. The device of claim 1, further comprising an insert (52), where the insert comprises a base (54) and three or more than three extensions (56) connected to the base and arranged radially around the base (54), and where each of the three or more than three extensions (56) is configured to fit within a corresponding fenestration (28) of the joint-ward end (16) of first section (12) of the device.

13. The device of claim 12 where the insert further comprises porous biological material impregnated with matrix-promoting substances or serves as a scaffold for progenitor cells, or comprises both porous biological material impregnated with matrix-promoting substances and serves as a scaffold for progenitor cells.

14. The device of claim 1, where the first section (12) and second section (14) are a unified whole and are not separable.

## Patentansprüche

1. Vorrichtung zur Linderung von Gelenkleiden und -erkrankungen, wobei die Vorrichtung Folgendes umfasst:
a) einen ersten Abschnitt (12), der ein gelenkseitiges Ende (16), ein entgegengesetztes Anschlussende (18) und eine Seitenwand (20), die sich zwischen dem gelenkseitigen Ende (16) und dem Anschlussende (18) erstreckt, umfasst;
wobei der erste Abschnitt (12) weiterhin eine Randsäule (22), die zum Teil die Seitenwand (20) des ersten Abschnitts (12) bildet, eine zentrale Säule (24) und drei oder mehr als drei Streben (26) umfasst, wobei jede Strebe sich zwischen der Randsäule (22) und der zentralen Säule (24) erstreckt und diese verbindet und jede Strebe dadurch die zentrale Säule (24) trägt;
wobei das gelenkseitige Ende (16) weiterhin mehrere Fenestrationen (28) umfasst, wobei jede Fenestration durch ein Zusammenlaufen der Randsäule (22), der zentralen Säule (24) und zwei benachbarten Streben (26) der drei oder mehr als drei Streben (26) gebildet wird; und
wobei der erste Abschnitt (12) weiterhin eine zentrale Öffnung (30) innerhalb der zentralen Säule (24), die von der zentralen Säule (24) gebildet wird, umfasst; und
b) einen zweiten Abschnitt (14), der ein Anschlussende (36), ein entgegengesetztes Vorderende (38) und eine Seitenwand (40), die sich zwischen dem Anschlussende (36) und dem Vorderende (38) erstreckt, umfasst;
**dadurch gekennzeichnet, dass** die Seitenwand (40) des zweiten Abschnitts (14) Gewinde (46) umfasst und die zentrale Öffnung innerhalb der zentralen Säule dazu konfiguriert ist, mit einem Treiber (128) zusammenzupassen.

2. Vorrichtung nach Anspruch 1, die weiterhin eine axiale Länge umfasst, und wobei die axiale Länge zwischen 5 mm und 30 mm liegt.

3. Vorrichtung nach Anspruch 1, wobei der erste Abschnitt (12) weiterhin einen Durchmesser zwischen 5 mm und 30 mm umfasst.

4. Vorrichtung nach Anspruch 1, wobei eine oder mehr als eine Fenestration (28) eine andere Größe, eine andere Form oder sowohl eine andere Größe als auch eine andere Form als eine oder mehr als eine andere Fenestration umfasst.

5. Vorrichtung nach Anspruch 1, wobei die Randsäule (22) eine oder mehr als eine Kerbe umfasst.

6. Vorrichtung nach Anspruch 1, wobei das gelenkseitige Ende (16) ein konvexes Profil, ein konkaves Profil oder ein gerades Profil umfasst, wie in einer perspektivischen Seitenschnittansicht zu sehen.

7. Vorrichtung nach Anspruch 1, wobei das gelenkseitige Ende (16) einen Krümmungsradius zwischen etwa 10 mm und 50 mm umfasst.

8. Vorrichtung nach Anspruch 1, wobei die Seitenwand (20) des ersten Abschnitts (12) ein allgemein konvexes Profil umfasst, wie in einer perspektivischen Seitenschnittansicht zu sehen, oder wobei der zweite Abschnitt weiterhin eine axiale Länge zwischen 5 mm und 30 mm umfasst.

9. Vorrichtung nach Anspruch 1, wobei die Seitenwand des zweiten Abschnitts (14) zylindrisch oder allgemein konisch ist, sich zwischen dem Anschlussende und dem Vorderende verjüngend, gegebenenfalls wobei die Seitenwand des zweiten Abschnitts sich zwischen 0,2 Grad und 15 Grad verjüngt.

10. Vorrichtung nach Anspruch 1, wobei das Anschlussende (18) des ersten Abschnitts (12) und das Anschlussende (36) des zweiten Abschnitts (14) durch einen Anschlussmechanismus zusammenpassen, der umkehrbar ist, oder
wobei das Vorderende (38) eine Wellenkante, eine Schrägkante oder eine Kombination davon umfasst.

11. Vorrichtung nach Anspruch 1, wobei die Seitenwand (40) des zweiten Abschnitts (14) weiterhin mehrere Fenestrationen (48) zwischen den Gewinden (46) umfasst; gegebenenfalls wobei die mehreren Fenestrationen (48) durch ein Zusammenlaufen des Anschlussendes des ersten Abschnitts (12) und des Anschlussendes des zweiten Abschnitts (14)) gebildet werden.

12. Vorrichtung nach Anspruch 1, die weiterhin einen Einsatz (52) umfasst, wobei der Einsatz eine Basis (54) und drei oder mehr als drei Ausläufer (56), die mit der Basis verbunden sind und radial um die Basis (54) angeordnet sind, umfasst, und wobei jeder der drei oder mehr als drei Ausläufer (56) dazu konfiguriert ist, in eine entsprechende Fenestration (28) des gelenkseitigen Endes (16) des ersten Abschnitts (12) der Vorrichtung zu passen.

13. Vorrichtung nach Anspruch 12, wobei der Einsatz weiterhin poröses biologisches Material umfasst, das mit matrixfördernden Substanzen imprägniert ist, oder als ein Gerüst für Vorläuferzellen dient oder sowohl poröses biologisches Material umfasst, das mit matrixfördernden Substanzen imprägniert ist, als auch als ein Gerüst für Vorläuferzellen dient.

14. Vorrichtung nach Anspruch 1, wobei der erste Abschnitt (12) und der zweite Abschnitt (14) ein einheitliches Ganzes sind und nicht trennbar sind.

## Revendications

1. Dispositif d'amélioration d'affections et de maladies articulaires, le dispositif comprenant :
a) une première section (12) comprenant une extrémité orientée vers l'articulation (16), une extrémité d'accouplement opposée (18), et une paroi latérale (20) s'étendant entre l'extrémité orientée vers l'articulation (16) et l'extrémité d'accouplement (18) ;
dans lequel la première section (12) comprend en outre une colonne périphérique (22) formant en partie la paroi latérale (20) de la première section (12), une colonne centrale (24), et trois ou plus de trois supports (26), chaque support s'étendant entre et reliant la colonne périphérique (22) et la colonne centrale (24), et chaque support soutenant ainsi la colonne centrale (24) ;
dans lequel l'extrémité orientée vers l'articulation (16) comprend en outre une pluralité de fenestrations (28), chaque fenestration étant formée par une confluence de la colonne périphérique (22), de la colonne centrale (24) et de deux supports adjacents (26) desdits trois ou plus de trois supports (26) ; et
dans lequel la première section (12) comprend en outre une ouverture centrale (30) à l'intérieur de et formée par la colonne centrale (24), et
b) une deuxième section (14) comprenant une extrémité d'accouplement (36), une extrémité avant opposée (38), et une paroi latérale (40) s'étendant entre l'extrémité d'accouplement (36) et l'extrémité avant (38) ;
**caractérisé en ce que** la paroi latérale (40) de la deuxième section (14) comprend des filetages (46) et **en ce que** l'ouverture centrale à l'intérieur de la colonne centrale est configurée pour s'accoupler avec un élément d'entraînement (128).

2. Dispositif selon la revendication 1, comportant en outre une longueur axiale, et dans lequel la longueur axiale est de 5 mm à 30 mm.

3. Dispositif selon la revendication 1, dans lequel la première section (12) comporte en outre un diamètre de 5 à 30 mm.

4. Dispositif selon la revendication 1, dans lequel une ou plusieurs fenestrations (28) ont une taille différente, une forme différente ou à la fois une taille différente et une forme différente de celle(s) d'une ou plusieurs autres fenestrations.

5. Dispositif selon la revendication 1, dans lequel la colonne périphérique (22) comprend un ou plusieurs crans.

6. Dispositif selon la revendication 1, dans lequel l'extrémité orientée vers l'articulation (16) comporte un profil convexe, un profil concave ou un profil rectiligne, vu en coupe transversale et en perspective latérale.

7. Dispositif selon la revendication 1, dans lequel l'extrémité orientée vers l'articulation (16) comporte un rayon de courbure d'environ 10 mm à 50 mm.

8. Dispositif selon la revendication 1, dans lequel la paroi latérale (20) de la première section (12) comporte un profil généralement convexe, vu en coupe transversale et en perspective latérale, ou dans lequel la deuxième section comporte en outre une longueur axiale de 5 mm à 30 mm.

9. Dispositif selon la revendication 1, dans lequel la paroi latérale de la deuxième section (14) est cylindrique ou généralement conique, s'amincissant entre l'extrémité d'accouplement et l'extrémité avant, optionnellement dans lequel la paroi latérale de la deuxième section s'amincit entre 0,2 degré et 15 degrés.

10. Dispositif selon la revendication 1, dans lequel l'extrémité d'accouplement (18) de la première section (12) et l'extrémité d'accouplement (36) de la deuxième section (14) s'accouplent au moyen d'un mécanisme d'accouplement réversible, ou
dans lequel l'extrémité avant (38) comporte un bord dentelé, un biseau, ou une combinaison de ceux-ci.

11. Dispositif selon la revendication 1, dans lequel la paroi latérale (40) de la deuxième section (14) comprend en outre une pluralité de fenestrations (48) entre les filetages (46) ; optionnellement dans lequel la pluralité de fenestrations (48) est formée par une confluence de l'extrémité d'accouplement de la première section (12) et de l'extrémité d'accouplement de la deuxième section (14).

12. Dispositif selon la revendication 1, comprenant en outre un insert (52), l'insert comprenant une base (54) et trois ou plus de trois prolongements (56) reliés à la base et disposés radialement autour de la base (54), et chacun desdits trois ou plus de trois prolongements (56) étant configuré pour s'insérer dans une fenestration correspondante (28) de l'extrémité orientée vers l'articulation (16) de la première section (12) du dispositif.

13. Dispositif selon la revendication 12, dans lequel l'insert comprend en outre un matériau biologique poreux imprégné de substances agissant favorablement sur la matrice ou sert d'échafaudage pour des cellules progénitrices, ou comprend un matériau biologique poreux imprégné de substances agissant favorablement sur la matrice tout en servant d'échafaudage pour des cellules progénitrices.

14. Dispositif selon la revendication 1, dans lequel la première section (12) et la deuxième section (14) sont un ensemble unifié et ne sont pas séparables.
